# EUROPEAN PATENT APPLICATION

(11) **EP 2 256 712 A1**
(43) Date of publication of application: **01.12.2010**
(21) Application number: 09722912.4
(22) Date of filing: 02.03.2009
(51) Int. Cl.: G09F 3/00, G09F 3/02, G09F 3/10, A61B 5/00, A61B 5/145, G01N 27/28, G01N 27/416

(54) **MEDICAL DEVICE**

(30) Priority: 21.03.2008 JP 2008073476
(71) Applicant: ARKRAY, Inc., Kyoto-shi, Kyoto 601-8045 (JP)
(72) Inventor: KUBO, Masayuki, Kyoto-shi Kyoto 601-8045 (JP)
(74) Representative: Mathys & Squire LLP
(86) International application number: PCT/JP2009/000929
(87) International publication number: WO 2009/116234

(57) **Abstract**

A medical device 1 measures a condition of a living body. The medical device 1 includes a main body 2 containing a measuring device inside, and a sheet member 3. The sheet member 3 displays information 4 on a user on one principal surface, with the other principal surface being attached to an outer surface of the main body 2. The sheet member 3 may include a sheet-like base 6, a protective layer 5 formed on one principal surface 6a of the base 6, and an adhesive material layer 7 formed on the other principal surface 6b of the base 3. At this time, it is preferable that the protective layer 5 is formed of a material having optical transparency, and that the information 4 on the user is displayed on the one principal surface 6a of the base 6.

## Description

### Technical Field

The present invention relates to medical devices, and particularly to a portable medical device.

### Background Art

Recently, the size reduction of medical devices has advanced, and the sizes of glucose meters, sphygmomanometers, pulse rate meters, and the like, for example, have been reduced to such a degree that they are portable. Among these, the glucose meter is an indispensable medical device for diabetic patients to always carry with them, and thus is a highly personal medical device.

Moreover, for being so highly personal, portable medical devices such as glucose meters are required to be identifiable by patients who use them from their exterior. Therefore Patent document 1, for example, discloses a protective cover that displays information such as a name of a patient. When the protective cover of Patent document 1 is attached to a medical device, the user of the medical device is easily identified from its exterior. Moreover, Patent document 2 discloses a protective cover similar to that of Patent document 1. It is expected that displaying names or the like of a patient on the protective cover disclosed in Patent document 2 also achieves the same advantages as the protective cover of the Patent document 1.
Patent document 1: JP2003-107092A (page 5, FIG. 2)
Patent document 2 JP2004-313269A (page 5, FIG. 6)

### Disclosure of Invention

### Problem to be Solved by the Invention

However, the protective covers of the above Patent documents 1 and 2 are configured to cover the entire front and side surfaces of a medical device, and a mold is required for manufacturing them. Therefore, the cost is high, resulting in an economic burden on patients. Moreover, the protective covers disclosed in the above Patent documents 1 and 2 are structurally voluminous, and thus prevent size reduction of medical devices.

Furthermore, the protective covers disclosed in the above Patent documents 1 and 2 are exclusively manufactured to suit the outer shape of a medical device to which they are attached. Thus when a patient changes to another medical device or to a new device, the protective cover needs to be changed as well accordingly, resulting in an economic burden on the patients also in this respect.

It is an object of the present invention to provide a medical device that solves the above-described problem, and displays information on a user on its exterior, while suppressing increase in cost and upsizing of the device.

### Means for Solving Problem

In order to achieve the above-described object, a medical device according to the present invention is a medical device that measures a condition of a living body, including a main body containing a measuring device inside, and a sheet-like member, the sheet-like member displaying information on a user on one principal surface, with the other principal surface being attached to an outer surface of the main body.

With a medical device according to the present invention, since the information is displayed on the sheet-like member, a protective cover as in conventional devices is not required. Moreover, the present invention is applicable regardless of the shape of the main body of the medical device. Thus with the present invention, increase in cost of a medical device is suppressed, and an economic burden on a patients is reduced. Moreover, with the present invention, increase in size of the medical device is also suppressed.

Moreover, in a preferred mode (a first mode) of the medical device according to the above-described present invention, either one or both of a projection and a recess is formed on the one principal surface of the sheet-like member, and the projection and the recess that are formed express information on operation of the medical device by means of at least one of arrangement, shape, and size thereof. According to the first mode, in particular, when the user is a patient with weak eyesight, the convenience of the patient is enhanced.

Moreover, in another preferred mode (a second mode) of the medical device according to the above-described present invention, the sheet-like member includes a storage device that exchanges information with the measuring device. According to the second mode, for example, a medical institution can manage patients, by collecting only the sheet-like member of the medical device used by the patient. Moreover, in this regard, getting the patient to attach a new sheet member to the medical device will enable the patient to continue measuring without leaving the medical device. This is particularly advantageous when the patient is a diabetic patient and the medical device is a glucose meter.

Furthermore, in another preferred mode (a third mode) of the medical device according to the above-described present invention, the sheet-like member includes a sheet-like base, a protective layer formed on one principal surface of the base, and an adhesive material layer formed on the other principal surface of the base, the protective layer being formed of a material having optical transparency. According to the third mode, durability of the printed information is enhanced.

Moreover, in the third mode, it is preferable that the sheet-like member has granular identification members that are dispersed inside the protective layer, the identification members being dispersed in such a manner that an area where the identification member exist does not overlap the information in a direction of the normal to the base, and being formed of any of a luminescent material, a thermochromic material, and a light reflective material. In this case, the information displayed on the sheet-like member is highly visible from outside, which facilitates grasping of the position of the information by the user. Moreover, this helps the user recognize the information in an environment where recognition of the information is difficult.

Furthermore, in the third mode, it is preferable that the sheet-like member further includes an identification layer between the base and the protective layer, the identification layer being formed of a luminescent material or a light reflective material. Also in this case, the information displayed on the sheet-like member is highly visible from outside, which facilitates grasping of the position of the information by the user. Moreover, this helps the user recognize the information in an environment where recognition of the information is difficult.

Moreover, in the third mode, it is preferable that the sheet-like member further includes an identification layer that covers the protective layer, the identification layer having an opening in a portion that overlaps the information in a thickness direction of the base, and being formed of any of a luminescent material, a thermochromic material, and a light reflective material. Also in this case, the information displayed on the sheet-like member is highly visible from outside, which facilitates grasping of the position of the information by the user. Moreover, this helps the user recognize the information in an environment where recognition of the information is difficult.

Moreover, in the third mode, it is also preferable that the sheet-like member has an electronic circuit that communicates with outside by radio transmission or optical communication. In this case, a medical institution can manage entering and leaving or position in a ward of patients (users). Moreover, since the medical institution can manage the movement history in the ward of the medical device of each patient, even if a patient leaves his or her own medical device somewhere, the place where he or she has left it can be immediately identified. Furthermore, the sheet-like member may be equipped with an electronic money function, and thus the convenience of the patient is enhanced.

Moreover, in the third mode, it is preferable that the sheet-like member further includes a fragrance component-impregnated layer containing a fragrance component, the fragrance component-impregnated layer being arranged above the one principal surface of the base, and the protective layer being formed so as to cover the fragrance component-impregnated layer and having a vent for diffusing the fragrance component outward. In this case, the user can be relaxed with the fragrance component. In particular, when the user is a patient suffering from a disease, the fragrance component can alleviate pain.

Furthermore, in the third mode, it is preferable that the sheet-like member further includes a pressure sensor that detects a pressure added to the protective layer, a speaker, and a circuit substrate, the circuit substrate being arranged on the one principal surface of the base, and including a signal processing circuit that drives the speaker based on a signal from the pressure sensor, and the protective layer being formed so as to cover the circuit substrate, the pressure sensor, and the speaker. In this case, a sound is outputted depending on pressing by a patient (user), which is particularly advantageous for a patient with a reduced sense of touch in his or her fingertips.

### Effects of the Invention

As described above, with the medical device according to the present invention, it is possible to display information on a user on its exterior, while suppressing increase in cost and suppressing upsizing of the device.

### Brief Description of Drawings

[FIG. 1] FIG. 1 is a perspective view illustrating a medical device according to Embodiment 1 of the present invention.
[FIG. 2] FIGS. 2(a) and 2(b) are diagrams illustrating a sheet-like member included in the medical device shown in FIG. 1. FIG. 2(a) is a plan view illustrating an upper surface of the sheet-like member, and FIG. 2(b) is a cross-sectional view illustrating a cross section taken along the cutting-plane line X-X' in FIG. 2(a).
[FIG. 3] FIG. 3 is a perspective view illustrating a medical device according to Embodiment 2 of the present invention.
[FIG. 4] FIGS. 4(a) and 4(b) are diagrams illustrating a sheet-like member included in the medical device shown in FIG. 3. FIG. 4(a) is a perspective view illustrating the sheet-like member three-dimensionally, and FIG. 4(b) is a cross-sectional view illustrating a cross section taken along the cutting-plane line Y-Y' in FIG. 4(a).
[FIG. 5] FIGS. 5(a) to 5(c) are diagrams illustrating other examples of a sheet-like member used in Embodiment 2 of the present invention. FIGS. 5(a) to 5(c) are perspective views each illustrating a different example three-dimensionally.
[FIG. 6] FIGS. 6(a) and 6(b) are diagrams illustrating another example of a sheet-like member used in Embodiment 2 of the present invention. FIG. 6(a) is a perspective view illustrating the example of the sheet-like member three-dimensionally, and FIG. 6(b) is a cross-sectional view illustrating a cross section taken along the cutting-plane line Z-Z' in FIG. 6(a).
[FIG. 7] FIG. 7 is a perspective view illustrating a medical device according to Embodiment 3 of the present invention.
[FIG. 8] FIG. 8 is a cross-sectional view illustrating a sheet-like member included in the medical device shown in FIG. 7. The cross section shown in FIG. 8 is a cross section taken along the cutting-plane line W-W' in FIG. 7.
[FIG. 9] FIG. 9 is a block diagram illustrating the internal configuration of the medical device shown in FIG. 7.
[FIG. 10] FIG. 10 is a plan view illustrating a sheet-like member used in Embodiment 4 of the present invention.
[FIG. 11] FIG. 11(a) is a cross-sectional view illustrating a cross section of the sheet-like member taken along the cutting-plane line A-A' in FIG. 10, and FIG. 11(b) is a cross-sectional view illustrating a cross section of the sheet-like member taken along the cutting-plane line B-B' in FIG. 10.
[FIG. 12] FIGS. 12(a) to 12(c) are cross-sectional views illustrating an example (first example) of a manufacturing method of a sheet-like member used in Embodiment 4 of the present invention, each illustrating a series of main steps.
[FIG. 13] FIGS. 13(a) to 13(c) are cross-sectional views illustrating another example (second example) of a manufacturing method of a sheet-like member used in Embodiment 4 of the present invention, each illustrating a series of main steps.
[FIG. 14] FIG. 14 is a plan view illustrating a sheet-like member used in Embodiment 5 of the present invention.
[FIG. 15] FIG. 15(a) is a cross-sectional view illustrating a cross section of the sheet-like member taken along the cutting-plane line C-C' in FIG. 14, and FIG. 15(b) is a cross-sectional view illustrating a cross section of the sheet-like member taken along the cutting-plane line D-D' in FIG. 14.
[FIG. 16] FIG. 16 is a plan view illustrating a sheet-like member used in Embodiment 6 of the present invention.
[FIG. 17] FIG. 17(a) is a cross-sectional view illustrating a cross section of the sheet-like member taken along the cutting-plane line E-E' in FIG. 16, and FIG. 17(b) is a cross-sectional view illustrating a cross section of the sheet-like member taken along the cutting-plane line F-F' in FIG. 16.
[FIG. 18] FIG. 18 is a cross-sectional view illustrating a sheet-like member used in Embodiment 7 of the present invention.
[FIG. 19] FIG. 19 is a cross-sectional view illustrating a sheet-like member used in Embodiment 8 of the present invention.
[FIG. 20] FIG. 20 is a plan view illustrating a sheet-like member used in Embodiment 9 of the present invention.
[FIG. 21] FIG. 21(a) is a cross-sectional view illustrating a cross section of the sheet-like member taken along the cutting-plane line G-G' in FIG. 20, and FIG. 21(b) is a cross-sectional view illustrating a cross section of the sheet-like member taken along the cutting-plane line H-H' in FIG. 20.

### [Description of Reference Numerals]

- 1: medical device (Embodiment 1)
- 2: main body
- 3: sheet member (Embodiment 1)
- 3a, 3b: principal surface of sheet member
- 4: information on user (patient)
- 5: protective layer
- 6: sheet-like base
- 6a, 6b: principal surface of sheet-like base
- 7: adhesive material layer
- 8: sensor insertion opening
- 9: sensor
- 10: display screen
- 11: medical device (Embodiment 2)
- 12: sheet member (Embodiment 2)
- 13: projection
- 14: sheet-like base
- 15: adhesive material layer
- 16a, 16b, 16c, 16d: projection
- 17: medical device (Embodiment 3)
- 18: sheet member (Embodiment 3)
- 18a: principal surface of sheet member
- 19: terminal
- 20: resin layer
- 21: sheet-like base
- 22: adhesive material layer
- 23: storage device
- 24: external electrode
- 25: conductance path
- 26: electrode pad
- 27: display unit
- 28: measuring device
- 29: management device
- 31: sheet member (Embodiment 4)
- 32: existence area
- 33: information display area
- 34: protective layer
- 35: sheet-like base
- 35a, 35b: principal surface of base
- 36: adhesive material layer
- 37: identification member
- 38: plate
- 41: sheet member (Embodiment 5)
- 42: identification layer
- 43: plate
- 44: protective layer
- 45: sheet-like base
- 45a, 45b: principal surface of base
- 46: adhesive material layer
- 51: sheet member (Embodiment 6)
- 52: identification layer
- 52a: opening
- 53: plate
- 54: protective layer
- 55: sheet-like base
- 55a, 55b: principal surface of base
- 56: adhesive material layer
- 61: sheet member (Embodiment 7)
- 62: IC chip
- 63: plate
- 64: protective layer
- 65: sheet-like base
- 65a, 65b: principal surface of base
- 66: adhesive material layer
- 71: sheet member (Embodiment 8)
- 72: fragrance component-impregnated layer
- 73: plate
- 74: protective layer
- 75: sheet-like base
- 75a, 75b: principal surface of base
- 76: adhesive material layer
- 77, 78: vent
- 79: separation layer
- 81: sheet member (Embodiment 9)
- 82: circuit substrate
- 83: plate
- 84: protective layer
- 85: sheet-like base
- 85a, 85b: principal surface of base
- 86: adhesive material layer
- 87: pressure sensor
- 88: speaker
- 89: through hole

### Best Mode for Carrying Out the Invention

### (Embodiment 1)

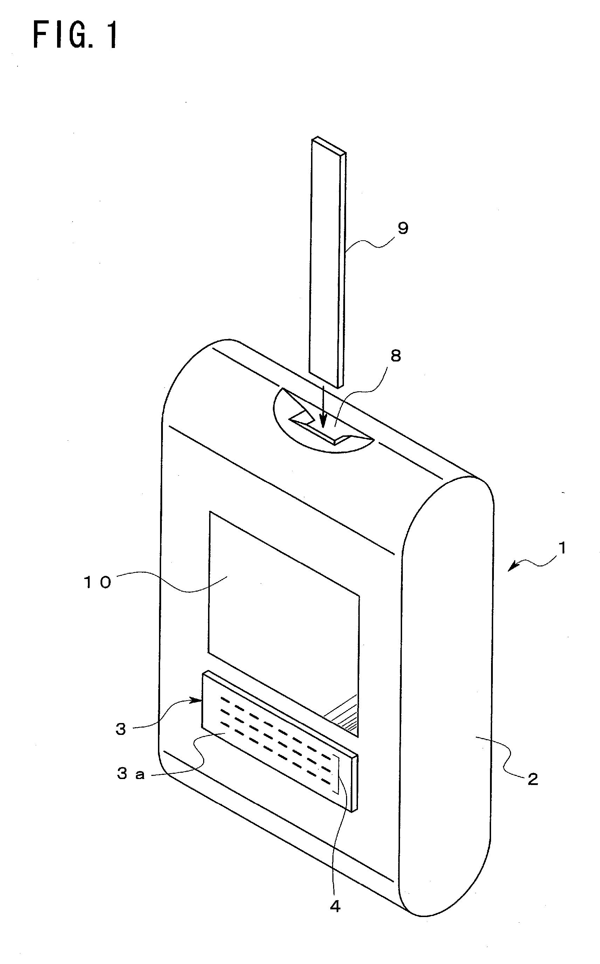

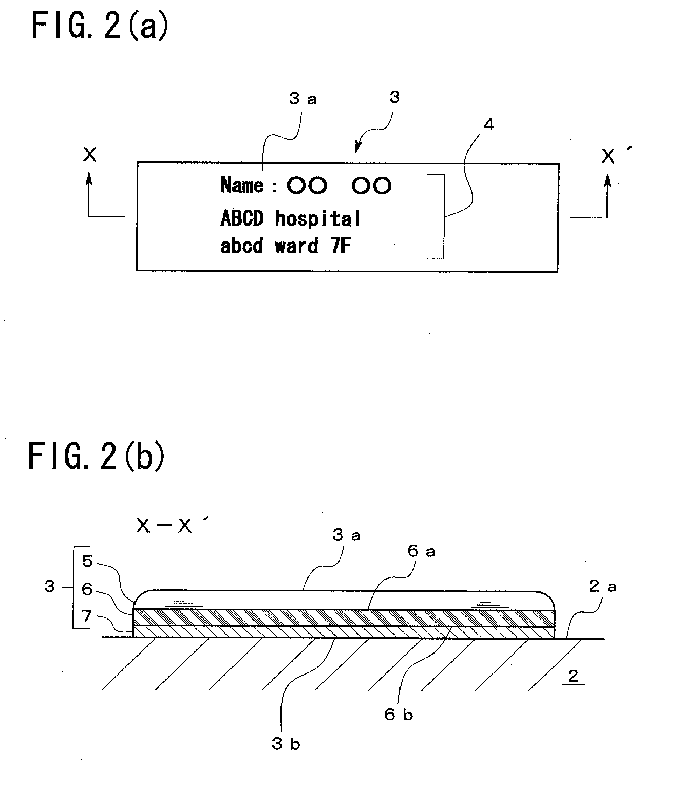

A medical device according to Embodiment 1 of the present invention is described referring to FIGS. 1 and 2. FIG. 1 is a perspective view illustrating a medical device according to Embodiment 1 of the present invention. FIGS. 2(a) and 2(b) are diagrams illustrating a sheet-like member included in the medical device shown in FIG. 1. FIG. 2(a) is a plan view illustrating an upper surface of the sheet-like member, and FIG. 2(b) is a cross-sectional view illustrating a cross section taken along the cutting-plane line X-X' in FIG. 2(a).

As shown in FIG. 1, a medical device 1 is a medical device that measures a condition of a living body, including, for example, a glucose meter, a sphygmomanometer, a lactate meter, a ketone body measuring device, a thermometer, a urine analyzer, and a lipid measuring device. Inside a main body 2 of the medical device 1, a measuring device (not shown in FIG. 1) according to the intended use is housed. Moreover, the medical device 1 is configured in a hand-held size, and is assumed to be carried by a user such as a patient, a doctor, and a nurse.

In Embodiment 1, the medical device 1 is a portable glucose meter that measures the blood glucose level of patient's blood. Moreover, in Embodiment 1, patient's blood is provided by a sensor 9, and the main body 2 includes a sensor insertion opening 8 for inserting the strip-like sensor 9. The sensor 9 contains a reagent inside, and the blood reacts with the reagent inside the sensor 9 beforehand. The measuring device inside the main body 2 has a function of measuring the blood glucose level from the blood that has reacted with the reagent by a colorimetric or electrochemical method. Measurement by the measuring device is started immediately after insertion of the sensor 9 into the sensor insertion opening 8. The measurement result is displayed on a display screen 10 provided for the main body 2.

Moreover, as shown in FIGS. 1, 2(a), and 2(b), the medical device 1 further includes a sheet-like member 3 (hereinafter referred to as "sheet member"), in addition to such conventional configuration. The sheet member 3 displays information 4 on a user on one principal surface 3 a, with the other principal surface 3b being attached to an outer surface 2a (see FIG. 2(b)) of the main body 2.

Examples of the information 4 on the user include the name of a patient who is the user, the ID number of the patient in a hospital, the name of the hospital that the patient visits, a responsible ward in the hospital, and the name of a doctor in charge. There is no particular limitation with respect to the information 4 on the user in the present embodiment.

Thus, the medical device 1 in Embodiment 1 displays the information such as the name of the patient outwardly from the device, without such a protective cover as disclosed in Patent documents 1 and 2. Moreover, the sheet member 3 can be manufactured at a lower cost than a protective cover disclosed in Patent documents 1 and 2. Furthermore, the sheet member 3 can be attached to any plane that has an equivalent area to its bottom, and therefore is easily applicable to the medical device 1 with a differently-shaped main body 2.

Therefore, according to the medical device 1 of Embodiment 1, increase in cost of a medical device is suppressed, and economic burden on a patient is reduced. Moreover, according to the medical device 1 of Embodiment 1, upsizing of a medical device is also suppressed. Furthermore, since the sheet member 3 employed in Embodiment 1 is not voluminous, unlike the protective cover disclosed in Patent documents 1 and 2, storing multiple sheets do not take a lot of space, and thus they are easily stored.

Moreover, in Embodiment 1, as shown in FIG. 2, the sheet member 3 may include a sheet-like base 6, a protective layer 5 formed on one principal surface 6a of the base 6, and an adhesive material layer 7 formed on the other principal surface 6b of the base 6. In this case, the information 4 on the user is displayed on the one principal surface 6a of the base 6 and is covered by the protective layer 5. Then, since the protective layer 5 is formed of a material having optical transparency, the information 4 on the user is displayed on the principal surface 3a of the sheet member 3.

Specifically, the protective layer 5 may be formed of a resin material or a rubber material. Examples of the resin material for forming the protective layer 5 include resin materials such as acrylate resins, urethane resins, polyethylene resins, polypropylene resins, ABS resins, polyvinyl chloride resins, AS resins, epoxy resins, and UV curable resins. Moreover, examples of the rubber material for forming the protective layer 5 include rubber materials such as silicone rubbers, butadiene rubbers, isoprene rubbers, chloroprene rubbers, butyl-rubbers, fluorocarbon rubbers, and styrene-butadiene rubbers.

As the base 6, when giving flexibility to the sheet member 3, it is preferable to use resin films such as a polyethylene film and a polycarbonate film, paper, or the like. When it is not required to give flexibility to the sheet member 3, a sheet formed of a material with little flexibility, such as metals, glasses, or hard resin materials may be used as the base 6. The information 4 on the user is displayed on the base 6 by printing with a laser printer, an ink-jet printer, and the like. The printed information 4 is protected by the protective layer 5, and thus is highly durable.

There is no particular limitation with respect to the formation material, the thickness, and the like of the adhesive material layer 7. As the formation material of the adhesive material layer 7, a material appropriately selected from the existing materials according to the formation material of the main body 2 may be used. Moreover, the thickness of the adhesive material layer 7 may be appropriately selected such that the desired adhesion is achieved. It should be noted that when the information 4 to be displayed needs to be changed, it is necessary to detach the sheet member 3, and therefore, it is preferable that the adhesive material layer 7 is formed such that its adhesion will not be too strong.

In Embodiment 1, the sheet member 3 is not limited to the examples shown in FIGS. 1, 2(a), and 2(b). For example, the sheet member 3 may be formed with the base 6 and the adhesive material layer 7 only. Moreover, the sheet member 3 may be attached to the outer surface 2a of the main body 2 with something other than an adhesive material, for example, a MAGICTAPE (registered trademark).

### (Embodiment 2)

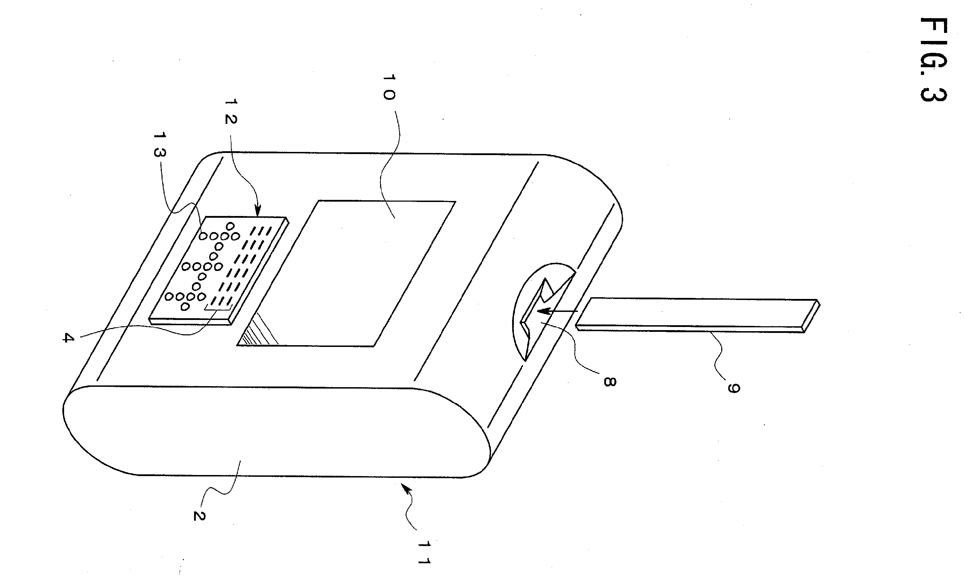

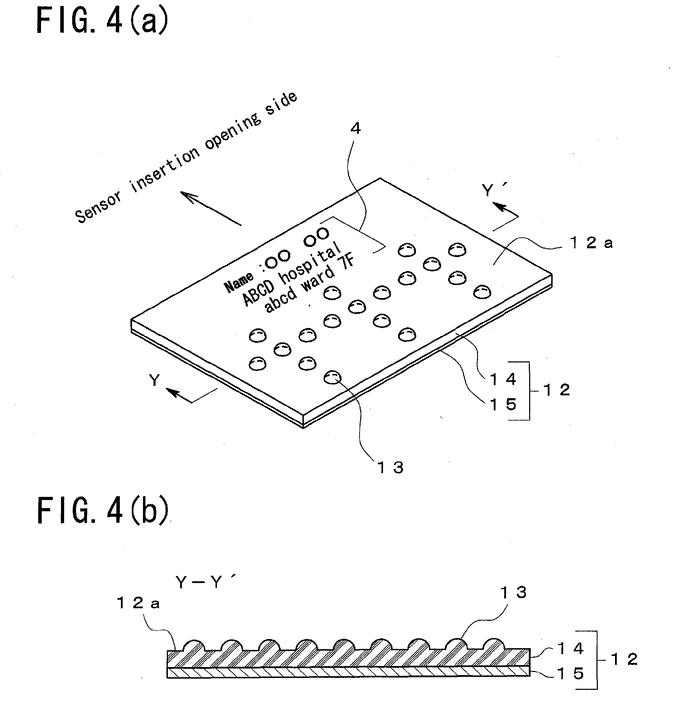

Next, a medical device according to Embodiment 2 is described referring to FIGS. 3 to 6. FIG. 3 is a perspective view illustrating a medical device according to Embodiment 2 of the present invention. FIGS. 4(a) and 4(b) are diagrams illustrating a sheet-like member included in the medical device shown in FIG. 3. FIG. 4(a) is a perspective view illustrating the sheet-like member three-dimensionally, and FIG. 4(b) is a cross-sectional view illustrating a cross section taken along the cutting-plane line Y-Y' in FIG. 4(a).

As shown in FIG. 3, a medical device 11 according to Embodiment 2 is a portable glucose meter, and has the same configuration as the medical device 1 according to Embodiment 1. However, the medical device 11 is different from the medical device 1 according to Embodiment 1 (see FIGS. 1 and 2) in the configuration of a sheet member 12. This is specifically described below.

As shown in FIGS. 3 and 4(a), in Embodiment 2, a projection 13 is formed on one principal surface 12a of the sheet member 12. Moreover, a plurality of projections 13 is formed to express information on operation of the medical device 11 by means of arrangement thereof. Specifically, as shown in FIG. 4(a), the plurality of projections 13 is arranged so that a user who touches it with his or her finger can perceive an arrow indicating an arbitrary direction. Then, the sheet member 12 is attached to the outer surface of the main body 2 in such a manner that the arrow formed by the projections 13 points toward the sensor insertion opening 8.

That is, in Embodiment 2, the information on operation of the medical device 1 is a position of the sensor insertion opening 8. The user can tell the position of the sensor insertion opening 8 from the sheet member 12. Thus, with Embodiment 2, the user, who is a patient, can tell the position of the sensor insertion opening 8 from a touch on the sheet member 12. The medical device 11 according to Embodiment 2 is particularly advantageous for a patient with weak eyesight, and the convenience of such a patient is enhanced.

Moreover, as shown in FIG. 4(b), in the sheet member 12, the projections 13 are integrally formed on the one principal surface of the sheet-like base 14. The adhesive material layer 15 is formed on the other principal surface of the base 14 on which no projection 13 is provided. It should be noted that the information 4 on the user is printed in an area of the one principal surface where the projections 13 are not formed.

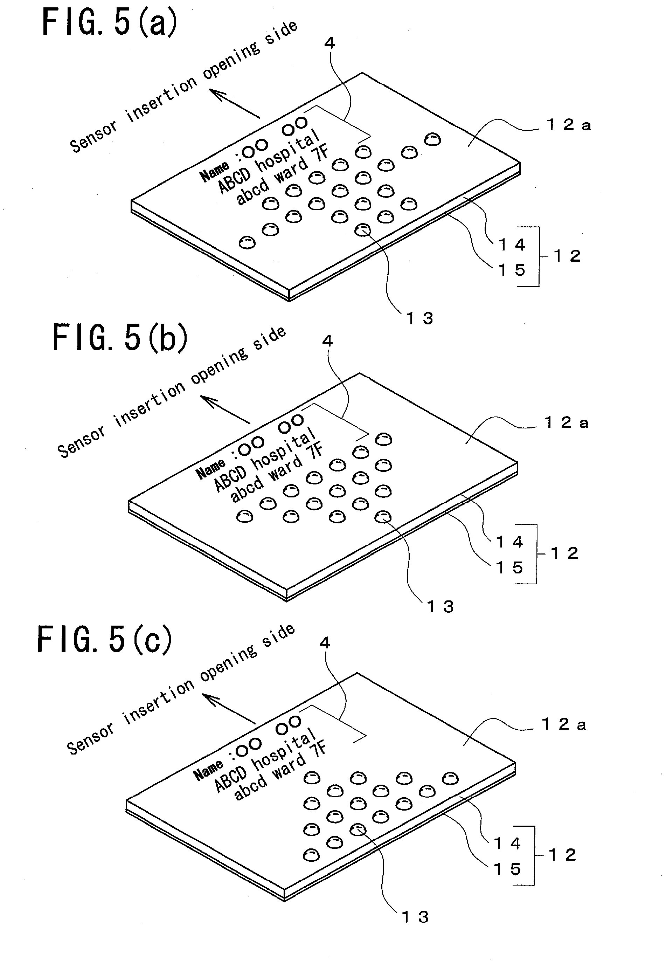

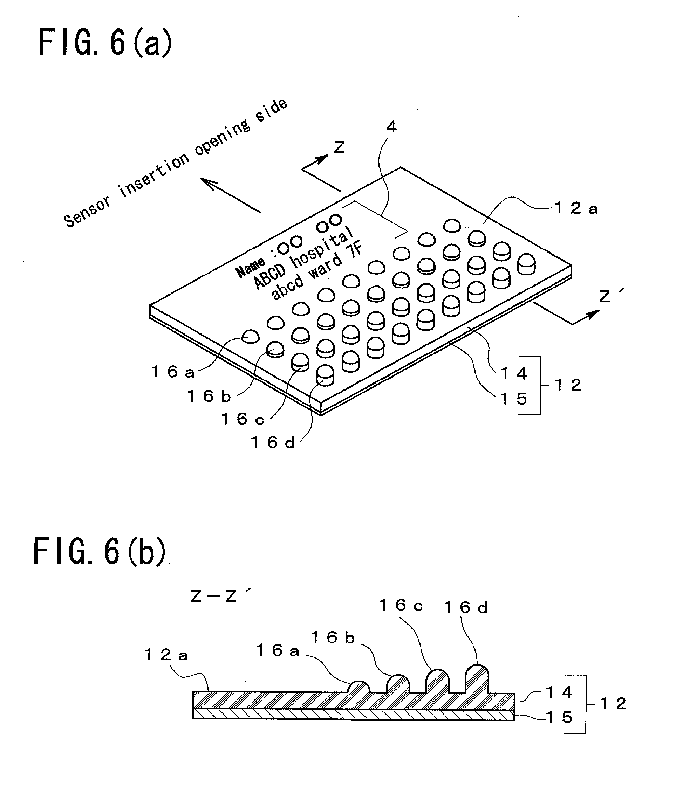

In Embodiment 2, the arrangement of the plurality of projections 13 is not limited to the mode shown in FIG. 4(a). Here, other examples of the sheet member 12 used in Embodiment 2 are described referring to FIGS. 5 and 6. FIGS. 5(a) to 5(c) are diagrams illustrating other examples of a sheet-like member used in Embodiment 2 of the present invention. FIGS. 5(a) to 5(c) are perspective views each illustrating a different example three-dimensionally.

Also in the examples shown in FIGS. 5(a) to 5(c), a user who touches the plurality of projections 13 can perceive an arbitrary direction. Therefore, also in the case where the sheet member 12 shown in FIGS. 5(a) to 5(c) is attached to the main body 2 (see FIG. 3), the user, who is a patient, can tell the position of the sensor insertion opening 8 from a touch on the sheet member 12.

Incidentally, in the examples shown in FIGS. 4(a) to 5(c), the information on operation of the medical device 11 is expressed by means of arrangement of the projections 13, but Embodiment 2 is not limited to these examples. The information on operation of the medical device 11 (the position of the sensor insertion opening 8) may be expressed by means of, for example, shape or size of the projection 13. Description is given in this respect referring to FIG. 6.

FIGS. 6(a) and 6(b), as with FIGS. 5(a) to 5(c), are diagrams illustrating another example of a sheet-like member used in Embodiment 2 of the present invention. FIG. 6(a) is a perspective view illustrating the example of the sheet-like member three-dimensionally, and FIG. 6(b) is a cross-sectional view illustrating a cross section taken along the cutting-plane line Z-Z' in FIG. 6(a).

As shown in FIGS. 6(a) and 6(b), in this example, information is expressed by means of the shape and the size of a projection. Specifically, in this example, four kinds of projections with different shapes and sizes, that is, projections 16a to 16d, are formed on the one principal surface 12a of the sheet member 12.

Specifically, the projection 16a is formed hemispherically, as with the projection 13 shown in FIGS. 4(a) to 5(c). The projections 16b to 16d have a shape obtained by combining a hemisphere and a cylinder, being different from each other in height of the cylinder-like part. The cylinder-like part of the projection 16b is the shortest, and that of the projection 16d is the tallest. Thus the projections 16a to 16d are different from each other in shape and size.

Then, the projections 16a to 16b are arranged in a line in the order of height. Moreover, multiple lines, each consisting of one each of projections 16a, 16b, 16c, and 16b, are provided in such a manner that they are parallel to each other and that projections having the same height lie side by side. Therefore, a user who touches the projections 16a to 16 can also perceive an arbitrary direction. Thus, also in the case where the sheet member 12 shown in FIGS. 6(a) and 6(b) is attached to the main body 2 (see FIG. 3), the user, who is a patient, can tell the position of the sensor insertion opening 8 from a touch on the sheet member 12.

In FIGS. 4(a) to 6(c), examples where projections are provided on the one principal surface 12a of the sheet member 12 are described, but Embodiment 2 is not limited to these examples. Embodiment 2 may employ a mode in which a recess is provided instead of a projection, or a mode in which a projection and a recess are provided. Also in these modes, the user can perceive information by a touch with his or her finger and the like.

Moreover, FIGS. 4(a) to 6(c) show an example of a projection that is circular in cross section perpendicular to its height, but in Embodiment 2, the cross section of a project or a recess is not limited to this example. Embodiment 2 may employ a mode in which a projection or a recess that is, for example, arrow-shaped or star-shaped in cross section perpendicular to its height is formed on the sheet member.

### (Embodiment 3)

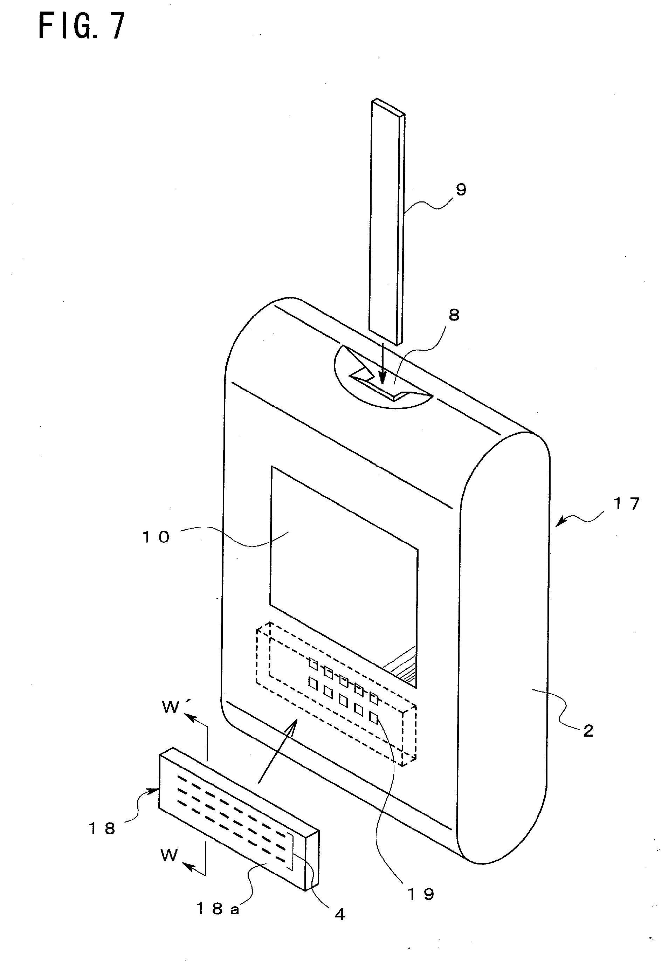

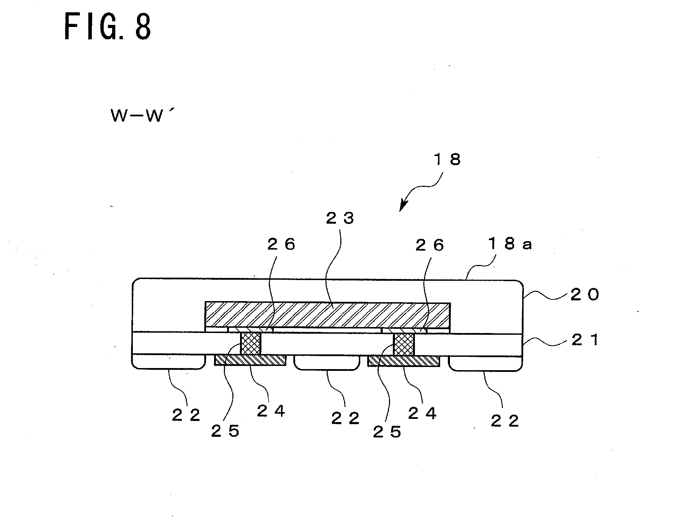

Next, a medical device according to Embodiment 3 is described referring to FIGS. 7 to 9. FIG. 7 is a perspective view illustrating a medical device according to Embodiment 3 of the present invention. FIG. 8 is a cross-sectional view illustrating a sheet-like member included in the medical device shown in FIG. 7. The cross section shown in FIG. 8 is a cross section taken along the cutting-plane line W-W' in FIG. 7. It should be noted that in FIG. 8, only the lines that appear in the cross section are shown.

As shown in FIG. 7, a medical device 17 according to Embodiment 3 is a portable glucose meter, and has the same configuration as the medical device 1 according to Embodiment 1. A sheet member 18, as with the sheet member 3 in Embodiment 1 (see FIGS. 1 and 2), displays information 4 on a user on one principal surface 18a.

However, in the medical device 17, the configuration of the sheet member 18 is different from that of the sheet member 3 according to Embodiment 1. Moreover, accordingly, the medical device 17 is also different from the medical device 1 according to Embodiment 1 (see FIGS. 1 and 2) in function of the measuring device included inside the main body 2 thereof. This is specifically described below.

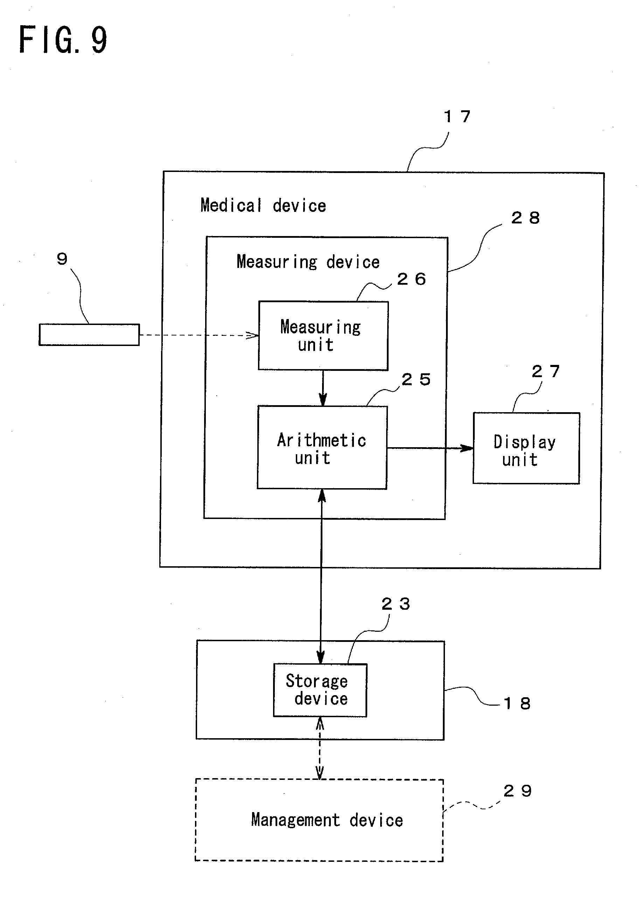

As shown in FIG. 8, in Embodiment 3, the sheet member 18 includes in its inside a storage device 23 that can exchange information with a measuring device 28 (see FIG. 9). In the storage device 23, electronic data of information 4 on a user is stored beforehand. Moreover, as shown in FIG. 7, in an area of the main body 2 of the medical device 17 to which the sheet member 18 is to be attached, a terminal 19 for connecting between the storage device 23 and the measuring device 28 inside the main body 2 is provided.

Specifically, as shown in FIG. 8, the sheet member 18 includes the storage device 23, a sheet-like base 21, a resin layer 20, and an adhesive material layer 22 for attaching the base 21. The resin layer 20 covers the base 21 together with the storage device 23. The information 4 on the user is printed on an outer surface of the resin layer 20. Moreover, the hatching of the resin layer 20, base 21, and adhesive material layer 22 is omitted in FIG. 8.

The base 21 includes a conductance path 25 that penetrates through it in a thickness direction. The conductance path 25 is formed by filling a through hole provided in the base 21 with a conductive material. It should be noted that a through hole having conductivity may be formed in the base 21, instead of the conductance path 25.

The storage device 23 is mounted on one principal surface of the base 21 in such a manner that its electrode pad 26 is in contact with one end of the conductance path 25. Moreover, on the other principal surface of the base 21, an external electrode 24 is formed so as to be in contact with the other end of the conductance path 25. Connecting the external electrode 24 to the terminal 19 provided for the main body 2 establishes exchange of information between the measuring device 28 (see FIG. 9) and the storage device 23. The adhesive material layer 22 is formed in an area of the principal surface of the base 21 where external electrode 24 is not formed.

Here, the configuration and function of the measuring device 28 according to this embodiment is described referring to FIG. 9. FIG. 9 is a block diagram illustrating the internal configuration of the medical device shown in FIG. 7. As shown in FIG. 9, the measuring device 28 includes a measuring unit 26 and an arithmetic unit 25. The measuring unit 26 obtains information for identifying a blood glucose level from the sensor 9.

For example, when an electrochemical method is employed as a measuring method by the measuring device 28, ferrocyan ion is generated by reaction between blood and reagent in the sensor 9. Then, the measuring unit 26 measures a current value of oxidation current of the ferrocyan ion, as information for identifying the blood glucose level. The measured current value is outputted from the measuring unit 26 to the arithmetic unit 25.

The arithmetic unit 25 calculates the blood glucose level from the measured current value. At this time, the blood glucose level is calculated by converting the current value to a blood glucose level with a calibration curve. The calibration curve is a function that indicates the relation between the current value and the blood glucose level, and has been obtained by experiment in advance. Moreover, the arithmetic unit 25 corrects the calculated blood glucose level with a correction value obtained by calibration. Then, the arithmetic unit 25 outputs data for display to a display unit 27, so that the calculated blood glucose level is displayed on the display screen 10 (see FIG. 7). The display unit 27 is a display device having a display screen 10.

Moreover, the arithmetic unit 25 outputs the information for identifying the calculated blood glucose level to the storage device 23. In Embodiment 3, the arithmetic unit 25 may output information for identifying the date of measurement, the calibration curve used for calculation, the correction value, the time of calibration, the manufacturer's serial number of the medical device 17 (or ID code set to the device), an error which has occurred at measurement, and the like. Furthermore, in a case where the medical device 17 has a fault diagnosis function, the arithmetic unit 25 may output the information on fault diagnosis to the storage device 23.

Thus, the storage device 23 can hold information on the medical device 17 including a measurement value, with output of information from the measuring device 28. In this case, for example, allowing a management device (a computer for management) 29 of a medical institution to acquire information from the storage device 23 will enable the medical institution to manage patients, by collecting only the sheet member 18 of the medical device 17 used by the patients. Moreover, in this regard, getting the patients to attach a new sheet member 18 to the medical device 17 will enable the patients to continue measuring without leaving the medical device 17.

Moreover, in a preferred mode of Embodiment 3, the medical institution can modify, for example, the calibration curve or correction value via the management device 29. Furthermore, in this case, it is preferable that when the storage device 23 holding the modified calibration curve or correction value is connected to the measuring device 28, the arithmetic unit 25 reads the modified calibration curve or correction value, and calculates with the information that has been read. In such a mode, improvement in measurement accuracy of the medical device 17 is further facilitated.

### (Embodiment 4)

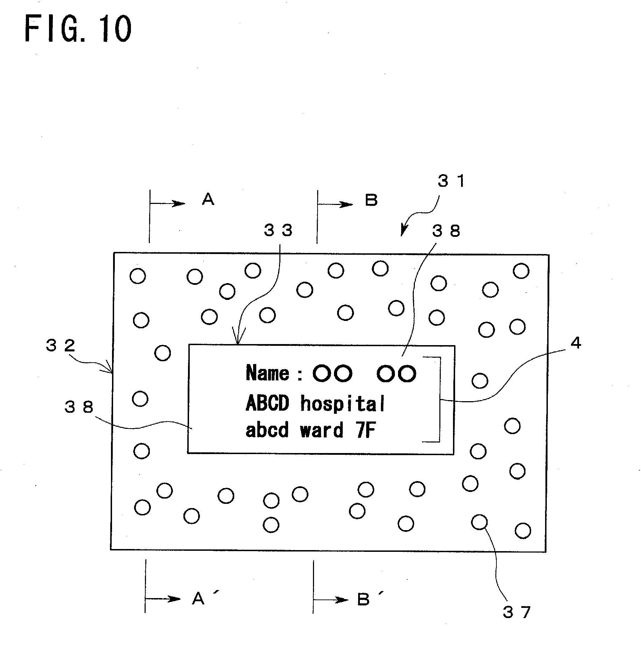

Next, a medical device according to Embodiment 4 is described referring to FIGS. 10 to 13. The medical device according to Embodiment 4 is configured in the same manner as the medical device according to Embodiment 1, 2, or 3, except that a sheet-like member (sheet member) 31 is differently configured. The configuration of the sheet member is mainly described below.

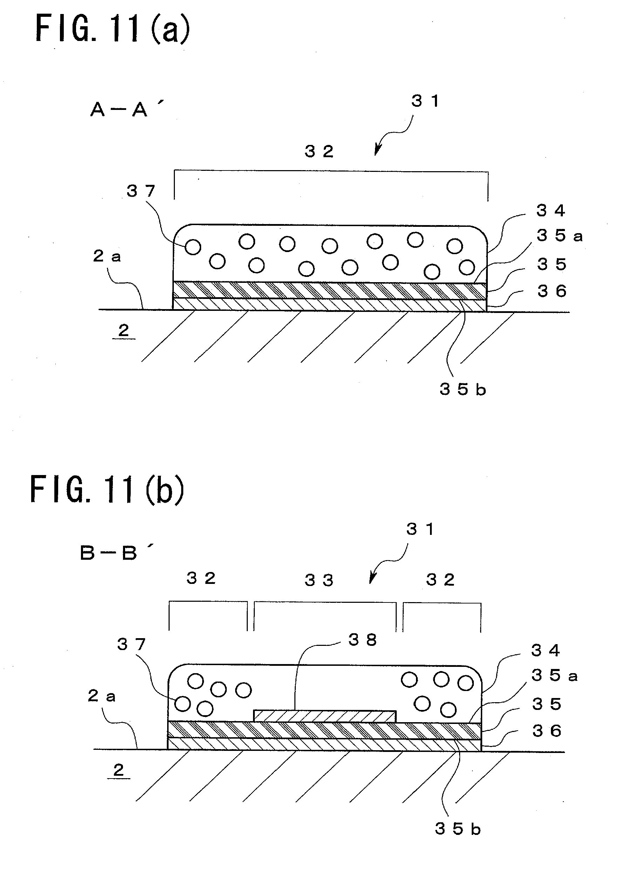

FIG. 10 is a plan view illustrating a sheet-like member used in Embodiment 4 of the present invention. FIG. 11(a) is a cross-sectional view illustrating a cross section of the sheet-like member taken along the cutting-plane line A-A' in FIG. 10, and FIG. 11(b) is a cross-sectional view illustrating a cross section of the sheet-like member taken along the cutting-plane line B-B' in FIG. 10. It should be noted that in FIGS. 11(a) and 11(b), only the lines that appear in the cross section are shown.

As shown in FIG. 10, in Embodiment 4, as with Embodiments 1 to 3, the sheet member 31 displays information 4 on a user on one principal surface, with the other principal surface being attached to an outer surface 2a of a main body of the medical device. Moreover, the sheet member 31 includes, as with the sheet member 3 in Embodiment 1 shown in FIG. 2(b), a sheet-like base 35, a protective layer 34 having optical transparency, and an adhesive material layer 36. Then, the protective layer 34 is formed on one principal surface (upper surface) 35a of the base 35, and the adhesive material layer 36 is formed on the other principal surface (lower surface) 35b of the base 35. Thus, the sheet member 31 has the same configuration as the sheet member 3 used in Embodiment 1 except for the following.

As shown in FIGS. 10, 11(a), and 11(b), in the sheet member 3, granular identification members 37 are dispersed inside the protective layer 34. Moreover, as shown in FIG. 10, the identification members 37 are dispersed in such a manner that an area where they exist (existence area) 32 surrounds the information 4 (that is, so as not to overlap the information 4) when viewed from above the sheet member 32 in the direction of the normal to the base 35. Moreover, in an area of the protective layer 34 that overlaps the information 4 (information display area) 33, the identification members 37 are not dispersed, so that identifying the information 4 from outside is not inhibited by the identification members 31.

Moreover, in Embodiment 4, the information 4 is printed on a surface of a plate 38 that is formed of paper, a resin material, or the like, and the plate 38 is arranged on the one principal surface 35a of the base 35. Therefore, an area that overlaps the printed surface of the plate 38 is the information display area 33 (see FIG. 11(b)). It should be noted that the hatching of the cross section of the protective layer 34 is omitted in FIGS. 11(a) and 11(b).

Furthermore, in Embodiment 4, the identification members 37 may be formed of a luminescent material, a thermochromic material, a light reflective material, and the like. Here, examples of the luminescent material include a phosphorescent material and a fluorescent material. Specific examples of the phosphorescent material include a material obtained by adding a rare-earth element such as europium (Eu) or dysprosium (Dy) to a crystal matrix formed of strontium aluminate (SrAl₂O₄), and a material obtained by adding copper (Cu) to zinc sulfide (ZnS). Examples of the fluorescent material include fluorescein isothiocyanate and tetramethyl rhodamine isothiocyanate.

Moreover, examples of the thermochromic material include a material containing a color former such as a leuco dye, a developer, and a color-changing-temperature controlling agent, wherein the color former and the developer bind to each other when the temperature has reached a certain temperature, thereby forming a color. A specific example thereof is metamo color (registered trademark) manufactured by PILOT Corporation. Examples of the light reflective material include metallic materials and glass materials.

Thus, in the medical device in Embodiment 4, the sheet member 31 is formed such that the information 4 displayed thereon is highly visible from outside because of the identification members 37. Therefore, even a user with weak eyesight can easily grasp the position of the information 4 because of the identification members 37 around the information 4. That is, in the sheet member 31, the identification members 37 help the user (particularly one with weak eyesight) grasp the position where the information 4 is disclosed.

Moreover, in the sheet member 31, the identification members 37 also have a function of helping the user recognize the information 4 in an environment where the user has difficulty in recognizing the information 4. Specifically, the identification members 37 being formed of the luminescent material or the light reflective material described above are advantageous when the user has to look for his or her own medical device in a relatively dark place. This is because light reflected or emitted from the identification members 37 help recognize the information in this case.

Moreover, in the case of using the thermochromic material described above as the formation material of the identification members 37, the identification members 37 detect the body temperature of the user or the ambient temperature, and change color. Then, by setting the temperatures at which the thermochromic material begins to change color to the upper limit (e.g., 40°C) and the lower limit (e.g., 10°C) of the usable temperature of the medical device, the user can determine whether the medical device is at a usable temperature based on the color of the identification members 37.

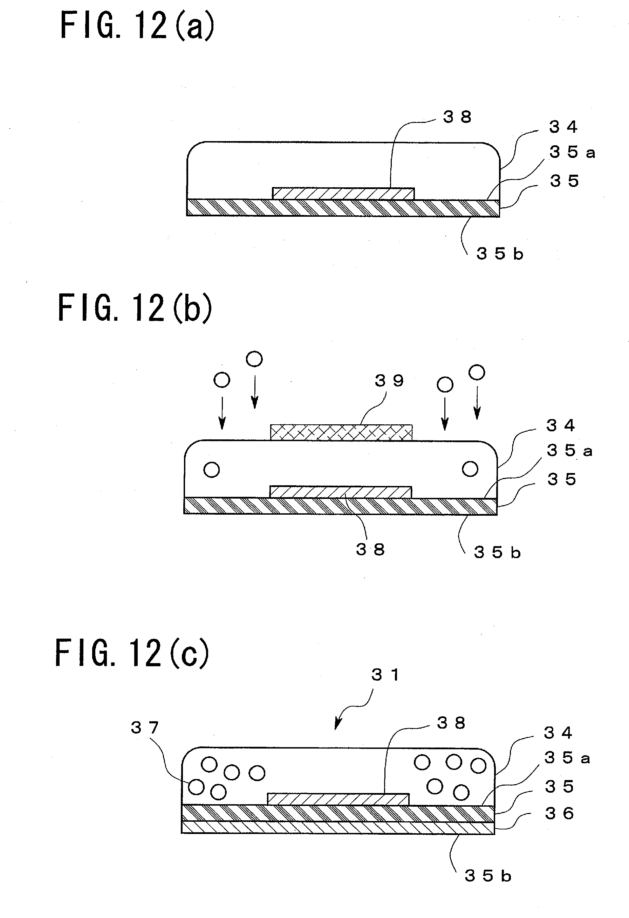

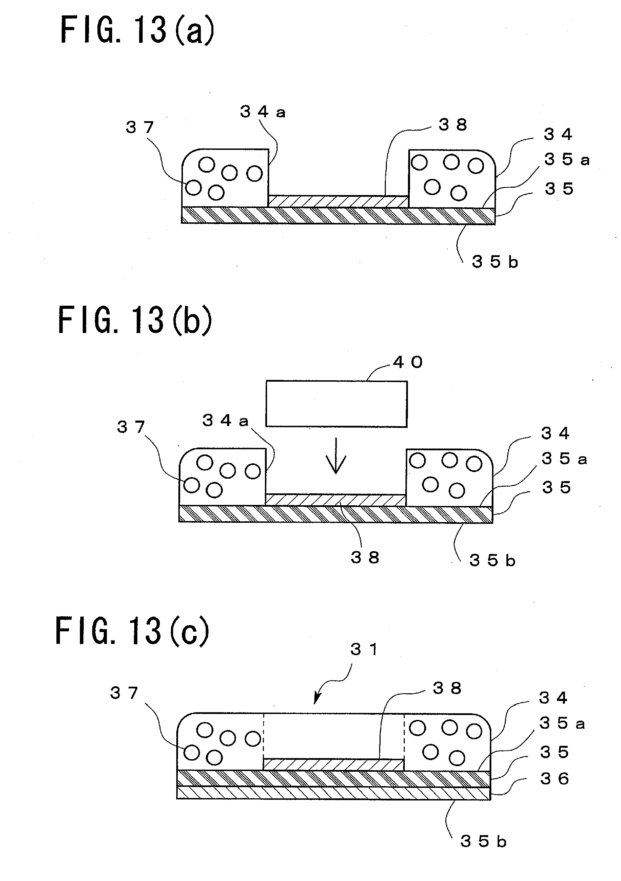

Here, a manufacturing method of the sheet member 31 is described referring to FIGS. 12 and 13. FIGS. 12(a) to 12(c) are cross-sectional views illustrating an example (first example) of the manufacturing method of a sheet-like member used in Embodiment 4 of the present invention, each illustrating a series of main steps. FIGS. 13(a) to 13(c) are cross-sectional views illustrating another example (second example) of the manufacturing method of a sheet-like member used in Embodiment 4 of the present invention, each illustrating a series of main steps.

The position where the cross sections shown in FIGS. 12(a) to 12(c) and 13 (a) to 13(c) are taken is the same as where the cross section shown in FIG. 11(b) is taken. Moreover, in FIGS. 12(a) to 12(c) and 13(a) to 13(c), only the lines that appear in the cross sections are shown.

Firstly, the first example is explained referring to FIG. 12. As shown in FIG. 12(a), the plate 38 on which the information 4 is printed is first arranged on the one principal surface 35a of the base 35, and subsequently, the protective layer 34 having optical transparency is formed so as to cover the plate 38. At this time, the protective layer 34 is formed by injecting a formation material fluidized by heating into a mold.

Next, as shown in FIG. 12(b), a mask 39 is arranged to cover the information display area 33, and then, with the protective layer 34 being heated, the granular identification members 37 are injected from above and dispersed within the protective layer 34. Subsequently, as shown in FIG. 12(c), after the protective layer 34 has become cold to be cured, the adhesive material layer 36 is formed on the other principal surface 35b of the base, so that the sheet member 31 is obtained.

Moreover, in the second example, as shown in FIG. 13(a), firstly, the protective layer 34 with the identification members 37 being dispersed therein is formed on the one principal surface 35a except for an area where the plate 38 is to be arranged, and subsequently, the plate 38 is placed. Specifically, a material with the identification members 37 being dispersed therein is injected into a mold that prevents the material from flowing into the area where the plate 38 is to be placed, so as to form the protective layer 34 provided with a recess 34a shown in FIG. 13(a).

Next, as shown in FIG. 13(b), a filling member 40 that conforms with the recess 34a is inserted in the recess 34a to form the protective layer 34 also above the plate 38. Subsequently, as shown in FIG. 13(c), the adhesive material layer 36 is formed on the other principal surface 35b of the base 35, so that the sheet member 31 is obtained.

It should be noted that the filling member 40 can be formed by melting the formation material of the protective layer 34 without the identification members 37 being dispersed therein, and injecting this material into a mold. Moreover, the formation material of the filling member 40 may be the same as the material injected in the step shown in FIG. 13(a), or may be different. When different, that is, when the material itself is different or when the material is different in degree of polymerization, and when a melting point of the material used in the step of FIG. 13(a) is higher than that of the formation material of the filling member 40, the filling member 40 is not required to be formed beforehand. In this case, the formation material of the filling member 40 may be injected into the recess 34a in a melted state.

Thus, the sheet member 31 shown in FIGS. 10 and 11 can be formed according to the forming method shown in FIGS. 12(a) to 12(c) or FIGS. 13(a) to (c). In Embodiment 4, the formation materials of the base 6 and the protective layer 5 of Embodiment 1 may be used as the formation materials of the base 35 and the protective layer 34. There is no particular limitation with respect to the formation material, the thickness, and the like of the adhesive material layer 36. Moreover, in Embodiment 4, the plate 38 does not need to be in touch with the base 35, but may be arranged off the base 35. For example, the plate 38 may be suspended within the protective layer 34. Furthermore, Embodiment 4 may employ a mode in which the sheet member 31 does not include the plate 38. In this case, the information 4 is printed on the one principal surface of the base 5.

### (Embodiment 5)

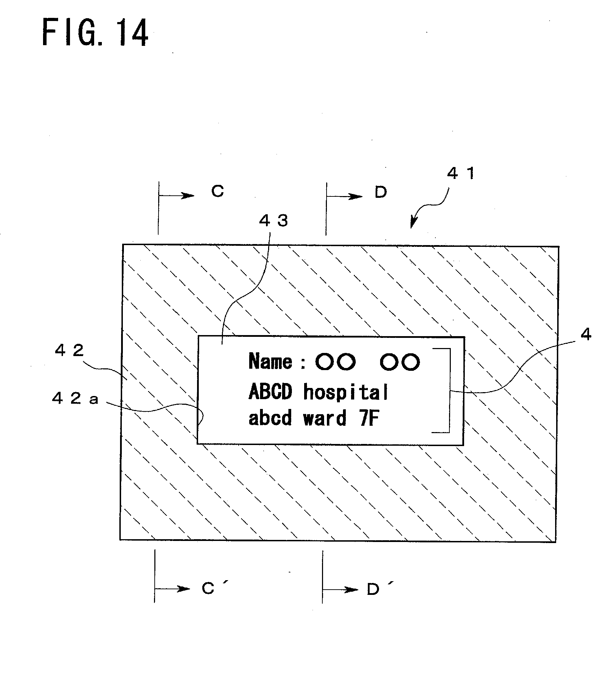

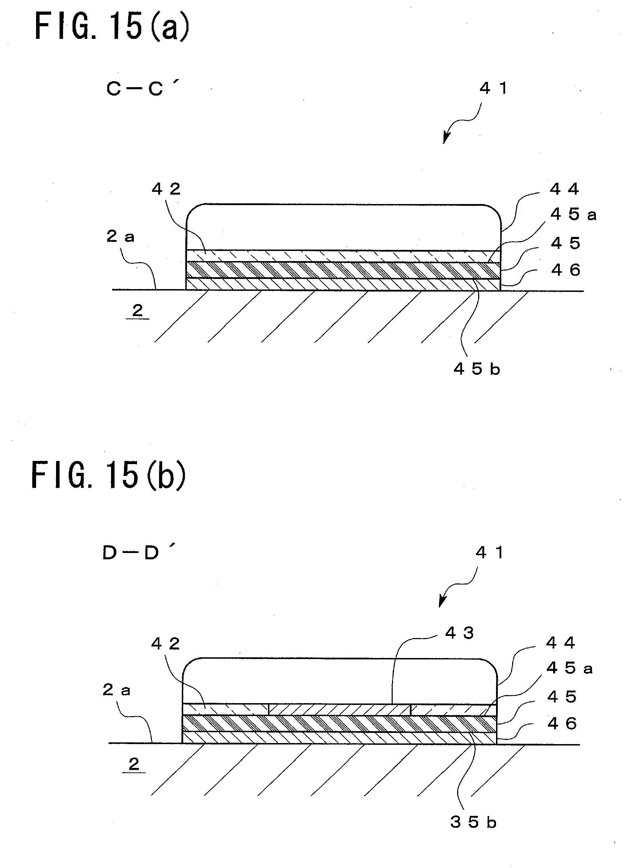

Next, a medical device according to Embodiment 5 is described referring to FIGS. 14 and 15. The medical device according to Embodiment 5 is also configured in the same manner as the medical device according to Embodiment 1, 2, or 3, except that a sheet-like member (sheet member) 41 is differently configured. The configuration of the sheet member is mainly described below.

FIG. 14 is a plan view illustrating a sheet-like member used in Embodiment 5 of the present invention. FIG. 15(a) is a cross-sectional view illustrating a cross section of the sheet-like member taken along the cutting-plane line C-C' in FIG. 14, and FIG. 15(b) is a cross-sectional view illustrating a cross section of the sheet-like member taken along the cutting-plane line D-D' in FIG. 14. It should be noted that in FIGS. 15(a) and 15(b), only the lines that appear in the cross section are shown.

As shown in FIG. 14, in Embodiment 5, as with Embodiments 1 to 3, the sheet member 41 displays information 4 on a user on one principal surface, with the other principal surface being attached to an outer surface 2a of a main body of the medical device. Moreover, the sheet member 41 includes, as with the sheet member 3 in Embodiment 1 shown in FIG. 2(b), a sheet-like base 45, a protective layer 44 having optical transparency, and an adhesive material layer 46. Then, the protective layer 44 is formed on one principal surface (upper surface) 45a of the base 45, and the adhesive material layer 36 is formed on the other principal surface (lower surface) 45b of the base 45. Thus, the sheet member 41 has the same configuration as the sheet member 3 used in Embodiment 1 except for the following.

As shown in FIGS. 14, 15(a) and 15(b), in Embodiment 5, the sheet member 41 includes an identification layer 42 and a plate 43 between the base 45 and the protective layer 44. As shown in FIG. 14, the plate 43 is arranged to be positioned approximately in the center of the one principal surface 45a of the base 45, and the information 4 is printed on its surface, as with the plate 38 of Embodiment 4 shown in FIG. 10.

Moreover, as shown in FIG. 14, the identification layer 42 is formed so as not to overlap the information 4 in thickness direction of the base 45, specifically, so as to surround the plate 43. In FIG. 14, an area where the identification layer 42 is formed is hatched. It should be noted that Embodiment 5 is not limited to the example shown in FIGS. 14, 15(a) and 15(b), but may be in a mode, for example, in which the identification layer 42 is formed to cover the entire one principal surface 45a of the base 45, and the plate 43 is arranged above the identification layer 42. Moreover, the hatching of the cross section of the protective layer 44 is omitted in FIGS. 15(a) and 15(b).

Moreover, the identification layer 42 is formed of a luminescent material, a light reflective material, or the like. Here, the luminescent materials in Embodiment 4 may be used as the luminescent material. However, in Embodiment 5, the luminescent material is shaped into a layer. Moreover, examples of the light reflective material include metallic materials and retro-reflective materials shaped into a layer. Specific examples of the metallic materials include aluminum and silver, and these materials are shaped into a foil or a thin film. Moreover, specific examples of the retro-reflective material include a sheet material in which a large number of glass beads having a diameter of approximately 50 to 500µm are arrayed on its surface.

Thus, in Embodiment 5, the information 4 displayed on the sheet member 41 is highly visible from outside because of the identification layer 42. Therefore, when Embodiment 5 is employed, as with Embodiment 4, even a user with weak eyesight can easily grasp the position of the information 4 because of the identification layer 42 around the information 4. That is, in the sheet member 41, the identification layer 42 helps the user (particularly one with weak eyesight) grasp the position where the information 4 is disclosed.

Moreover, the identification layer 42 of the sheet member 41 also has a function of helping the user recognize the information 4 in an environment where the user has difficulty in recognizing the information 4, as with the identification members 37 (see FIG. 10) in Embodiment 4. Specifically, the identification layer 42 being formed of the luminescent material or the light reflective material described above is advantageous when the user has to look for his or her own medical device in a relatively dark place. This is because light reflected or emitted from the identification layer 42 helps recognize the information in this case.

Also in Embodiment 5, the formation materials of the base 6 and the protective layer 5 of Embodiment 1 may be used as the formation materials of the base 45 and the protective layer 44. There is no particular limitation with respect to the formation material, the thickness, and the like of the adhesive material layer 46. Moreover, Embodiment 5 may employ a mode in which the sheet member 41 does not include the plate 43. In this case, the information 4 is printed on the one surface of the base 45, and the identification layer 42 is formed so as to surround the information 4 printed on the base 45.

### (Embodiment 6)

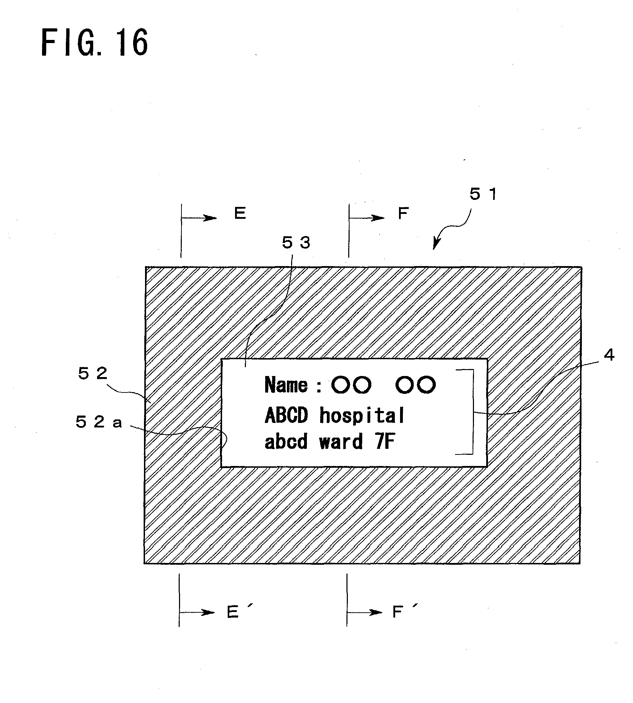

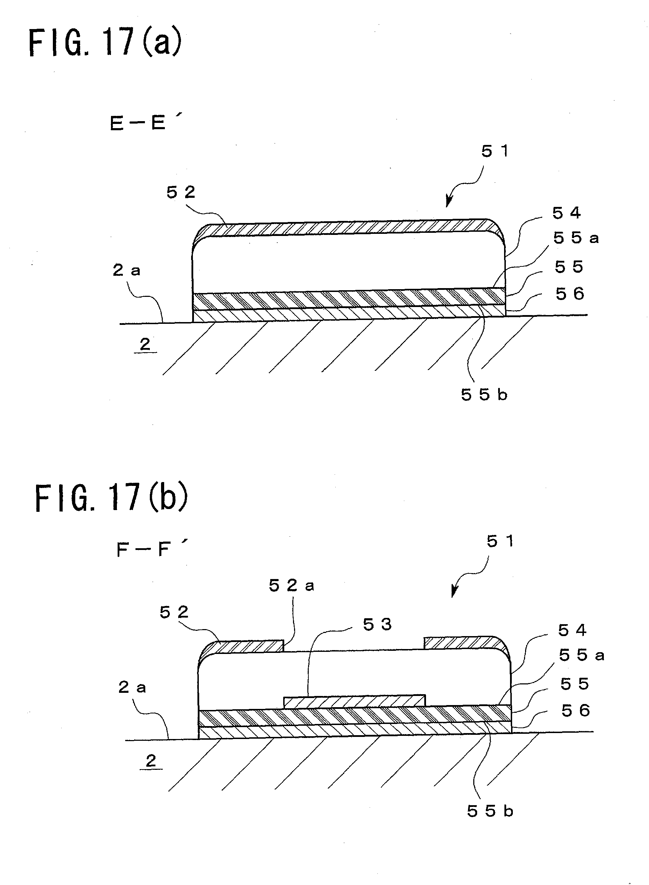

Next, a medical device according to Embodiment 6 is described referring to FIGS. 16 and 17. The medical device according to Embodiment 6 is also configured in the same manner as the medical device according to Embodiment 1, 2, or 3, except that a sheet-like member (sheet member) 51 is differently configured. The configuration of the sheet member is mainly described below.

FIG. 16 is a plan view illustrating a sheet-like member used in Embodiment 6 of the present invention. FIG. 17(a) is a cross-sectional view illustrating a cross section of the sheet-like member taken along the cutting-plane line E-E' in FIG. 16, and FIG. 17(b) is a cross-sectional view illustrating a cross section of the sheet-like member taken along the cutting-plane line F-F' in FIG. 16. It should be noted that in FIGS. 17(a) and 17(b), only the lines that appear in the cross section are shown.

As shown in FIGS. 16, 17(a), and 17(b), in Embodiment 6, as with Embodiments 1 to 3, the sheet member 51 displays information 4 on a user on one principal surface, with the other principal surface being attached to an outer surface 2a of a main body of the medical device. Moreover, the sheet member 51 includes, as with the sheet member 3 in Embodiment 1 shown in FIG. 2(b), a sheet-like base 55, a protective layer 54 having optical transparency, and an adhesive material layer 56. Then, the protective layer 54 is formed on one principal surface (upper surface) 55a of the base 55, and the adhesive material layer 56 is formed on the other principal surface (lower surface) 55b of the base 55. Thus, the sheet member 51 has the same configuration as the sheet member 3 used in Embodiment 1 except for the following.

As shown in FIGS. 16, 17(a) and 17(b), in Embodiment 6, the sheet member 51 includes an identification layer 52 that covers the protective layer 54, and a plate 53. As shown in FIG. 16, the plate 53 is arranged to be positioned approximately in the center of the one principal surface 55a of the base 55, and the information 4 is printed on its surface, as with the plate 38 of Embodiment 4 shown in FIG. 10. Moreover, the identification layer 52 is provided with an opening 52a in a portion that overlaps the information 4 in a thickness direction of the base 55. Therefore, the information 4 printed on the surface of the plate 53 is observable from outside via the opening 52a. It should be noted that the hatching of the cross section of the protective layer 54 is omitted in FIGS. 17(a) and 17(b).

The identification layer 52 is formed of a luminescent material, a light reflective materials, or the like, as with the identification layer 42 of Embodiment 5 shown in FIG. 14. Also in Embodiment 6, the luminescent materials in Embodiment 4 may be used as the luminescent material, as with the Embodiment 5. Moreover, examples of the light reflective material include metallic materials and retro-reflective materials shaped into a layer, as with Embodiment 5.

Thus, in Embodiment 6, the information 4 displayed on the sheet member 51 is highly visible from outside because of the identification layer 52. Therefore, when Embodiment 6 is employed, as with Embodiments 4 and 5, even a user with weak eyesight can easily grasp the position of the information 4 because of the identification layer 52 around the information 4. That is, in the sheet member 51, the identification layer 52 helps the user (particularly one with weak eyesight) grasp the position where the information 4 is disclosed.

Moreover, the identification layer 52 of the sheet member 51 also has a function of helping the user recognize the information 4 in an environment where the user has difficulty in recognizing the information 4, as with the identification members 37 (see FIG. 10) in Embodiment 4. Specifically, the identification layer 52 being formed of the luminescent material or the light reflective material described above is advantageous when the user has to look for his or her own medical device in a relatively dark place. This is because light reflected or emitted from the identification layer 52 helps recognize the information in this case.

Furthermore, in Embodiment 6, the identification layer 52 may be formed of the thermochromic material described in Embodiment 4, and also in this case, the effect described above is produced. Moreover, in this case, the identification layer 52 detects the body temperature of the user or the ambient temperature, and changes color. Then, by setting the temperatures at which the thermochromic material begins to change color to the upper limit and the lower limit of the usable temperature of the medical device, the user can determine whether the medical device is within a usable temperature based on the color of the identification layer 52.

Moreover, in Embodiment 6, a sheet-like thermometer may be used as the identification layer 52. Specific examples of the sheet-like thermometer include a liquid crystal thermometer including a cholesteric liquid crystal that changes color depending on the temperature. The liquid crystal thermometer can inform the user of approximate temperatures such as 10°C, 15°C, 20°C, 25°C, 30°C, 35°C, and 40°C. Thus, also in this case, as with the case where the identification layer 52 is formed of the thermochromic material, the user can determine whether the medical device is within a usable temperature range.

In Embodiment 6, the formation materials of the base 6 and the protective layer 5 of Embodiment 1 may be used as the formation materials of the base 55 and the protective layer 54. There is no particular limitation with respect to the formation material, the thickness, and the like of the adhesive material layer 56. Moreover, in Embodiment 6, the plate 53 does not need to be in touch with the base 55, but may be arranged off the base 55. For example, the plate 53 may be suspended within the protective layer 54. Moreover, Embodiment 6 may employ a mode in which the sheet member 51 does not include the plate 53. In this case, the information 4 is printed on the one principal surface of the base 55.

### (Embodiment 7)

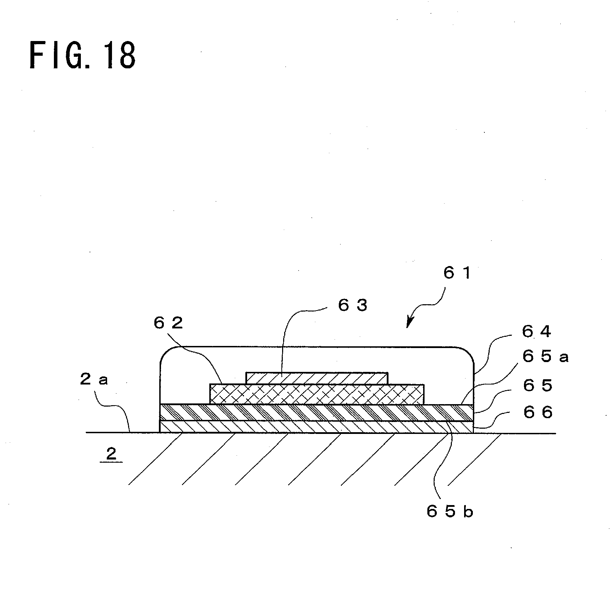

Next, a medical device according to Embodiment 7 is described referring to FIG. 18. The medical device according to Embodiment 7 is also configured in the same manner as the medical device according to Embodiment 1, 2, or 3, except that a sheet-like member (sheet member) 61 is differently configured. The configuration of the sheet member is mainly described below.

FIG. 18 is a cross-sectional view illustrating a sheet-like member used in Embodiment 7 of the present invention. The position where the cross section shown in FIG. 18 is taken is the same as where the cross sections shown in FIGS. 11(b), 15(b), and 17(b) are taken, and the cross section shown in FIG. 18 is taken along the cutting-plane line parallel to a short side of a rectangular sheet member. Moreover, in FIG. 18, only the lines that appear in the cross section are shown.

As shown in FIG. 18, in Embodiment 7, as with Embodiments 1 to 3, the sheet member 61 displays information (not shown in FIG. 18) on a user on one principal surface, with the other principal surface being attached to an outer surface 2a of a main body of the medical device. Moreover, the sheet member 61 includes, as with the sheet member 3 in Embodiment 1 shown in FIG. 2(b), a sheet-like base 65, a protective layer 64 having optical transparency, and an adhesive material layer 66. Then, the protective layer 64 is formed on one principal surface (upper surface) 65a of the base 65, and the adhesive material layer 66 is formed on the other principal surface (lower surface) 65b of the base 65. Thus, the sheet member 61 has the same configuration as the sheet member 3 used in Embodiment 1 except for the following.

As shown in FIG. 18, in Embodiment 7, the sheet member 61 includes an IC chip 62 arranged on one principal surface 65a of the base 65 and a plate 63 arranged on an upper surface of the IC chip 62. The information 4 is printed on a surface of the plate 63, as with the plate 38 of Embodiment 4 shown in FIG. 10. It should be noted that the hatching of the cross section of the protective layer 64 is omitted in FIG. 18.

The IC chip 62 is an IC chip that realizes an RF tag used for RFID (Radio Frequency Identification), and has an electronic circuit that communicates with the outside by radio transmission. Specifically, the IC chip 62 includes at least an antenna unit that transmits and receives information to and from an external reader/writer, a storage unit that holds the information, and a control unit that encodes and decodes the information, although this is not shown. It should be noted that transmission and reception of the information between the IC chip 62 and the outside is not limited to radio wave, but may be optical communication such as infrared and visible light.

With such a configuration, in Embodiment 7, the sheet member 61 is capable of using an IC tag for RFID. For example, with a medical device in Embodiment 7, a medical institution can manage entering and leaving or position in a ward of patients (users). Moreover, since the medical institution can manage the movement history in the ward of the medical device of each patient, even if a patient leaves his or her own medical device somewhere, the place where he or she has left it can be immediately identified. Furthermore, the sheet member 61 may be equipped with an electronic money function, and thus the convenience of the patient is enhanced.

In Embodiment 7, the formation materials of the base 6 and the protective layer 5 of Embodiment 1 may be used as the formation materials of the base 65 and the protective layer 64. There is no particular limitation with respect to the formation material, the thickness, and the like of the adhesive material layer 66. Moreover, in Embodiment 7, the plate 63 does not need to be in touch with the IC chip 62, but may be arranged off the IC chip 62. For example, the plate 63 may be suspended within the protective layer 64. Furthermore, Embodiment 7 may employ a mode in which the sheet member 61 does not include the plate 63. In this case, the information is printed on the upper surface of the IC chip 62.

### (Embodiment 8)

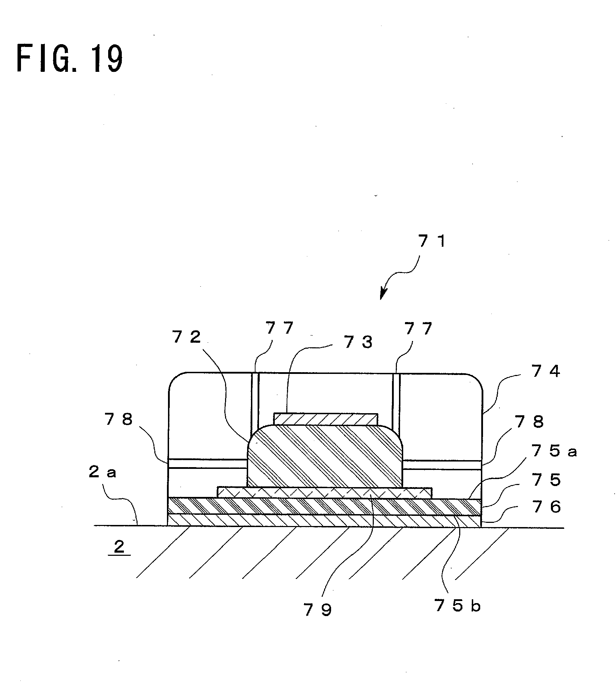

Next, a medical device according to Embodiment 8 is described referring to FIG. 19. The medical device according to Embodiment 8 is also configured in the same manner as the medical device according to Embodiment 1, 2, or 3, except that a sheet-like member (sheet member) 71 is differently configured. The configuration of the sheet member is mainly described below.

FIG. 19 is a cross-sectional view illustrating a sheet-like member used in Embodiment 8 of the present invention. The position where the cross sections shown in FIG. 19 is taken is the same as where the cross sections shown in FIGS. 11(b), 15(b), and 17(b) are taken, and the cross section shown in FIG. 19 is taken along the cutting-plane line parallel to a short side of a rectangular sheet member. Moreover, in FIG. 19, only the lines that appear in the cross section are shown.

As shown in FIG. 19, in Embodiment 8, as with Embodiments 1 to 3, the sheet member 71 displays information (not shown in FIG. 19) on a user on one principal surface, with the other principal surface being attached to an outer surface 2a of a main body of the medical device. Moreover, the sheet member 71 includes, as with the sheet member 3 in Embodiment 1 shown in FIG. 2(b), a sheet-like base 75, a protective layer 74 having optical transparency, and an adhesive material layer 76. Then, the protective layer 74 is formed on one principal surface (upper surface) 75a of the base 75, and the adhesive material layer 76 is formed on the other principal surface (lower surface) 75b of the base 75. Thus, the sheet member 71 has the same configuration as the sheet member 3 used in Embodiment 1 except for the following.

In Embodiment 8, the sheet member 71 includes a fragrance component-impregnated layer 72 impregnated with a fragrance component, a plate 73, and a separation layer 79, which are covered by a protective layer 74. The plate 73 is arranged above the fragrance component-impregnated layer 72, and the information 4 is printed on its surface, as with the plate 38 of Embodiment 4 shown in FIG. 10. It should be noted that the hatching of the cross section of the protective layer 74 is omitted in FIG. 19.

Furthermore, if the fragrance component-impregnated layer 72 is completely covered by the protective layer 74, the fragrance component contained therein is not diffused outward, and thus the protective layer 74 is provided with vents 77 and 78. The separation layer 79 is provided so that the fragrance component contained in the fragrance component-impregnated layer 72 does not exude to the base 75 or the adhesive material layer 76. Specifically, as the separation layer 79, it is preferable to use a metallic foil such as an aluminum foil.

The fragrance component-impregnated layer 72 is formed by impregnating a porous material with a material containing a fragrance component. Examples of the porous material include porous materials obtained by using the resin materials and rubber materials described as the formation materials of the protective layer 5 of Embodiment 1 as the starting materials, and foaming these materials.

Examples of the material containing a fragrance component include orange oil, lemon oil, lime oil, petitgrain oil , citrus junos oil, neroli oil, bergamot oil, lavender oil, lavandin oil, abies oil, bay oil, ylang-ylang oil, labdanum oil, citronella oil, geranium oil, peppermint oil, spearmint oil, eucalyptus oil, lemongrass oil, patchouli oil, jasmine oil, rose oil, cedar oil, vetiver oil, galbanum oil, oakmoss oil, pine oil, camphor oil, wood sandal oil, linalool, geraniol, citronellol, 1-menthol, borneol, β-phenylethyl alcohol, n-nonyl aldehyde, citral, cinnamic aldehyde, lilial, vanillin, camphor, ionone, ethylene brassylate, galaxolid, and linalyl acetate.

With this configuration, when a user, for example, presses the sheet member 71, the fragrance component diffuses via the through hole 77 or 78 provided for the sheet member 71. Then, the user can be relaxed with the fragrance component. In particular, when the user is a patient suffering from a disease, the fragrance component can be expected to alleviate pain. Furthermore, if the user is allowed to select the fragrance, the user can identify his or her own medical device by the fragrance from the sheet member 71.

In Embodiment 8, the formation materials of the base 6 and the protective layer 5 of Embodiment 1 may be used as the formation materials of the base 75 and the protective layer 74. There is no particular limitation with respect to the formation material, the thickness, and the like of the adhesive material layer 76. Moreover, in Embodiment 8, the plate 73 does not need to be in touch with the fragrance component-impregnated layer 72, but may be arranged off the fragrance component-impregnated layer 72. For example, the plate 73 may be suspended within the protective layer 74.

### (Embodiment 9)

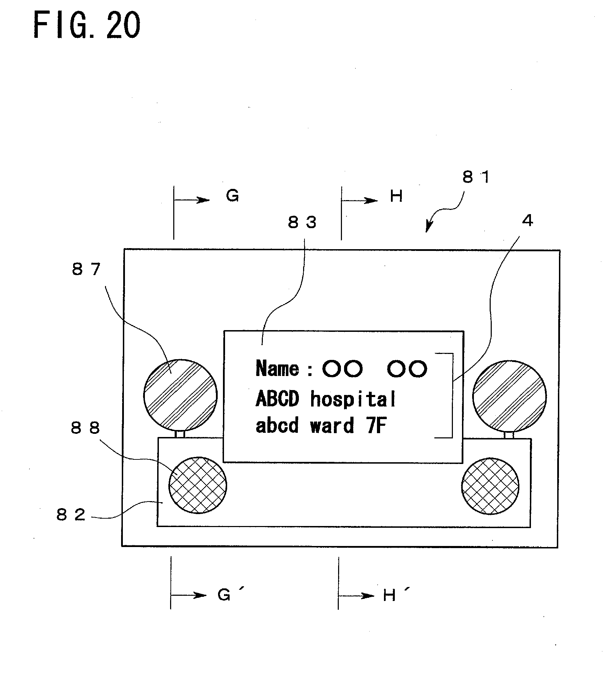

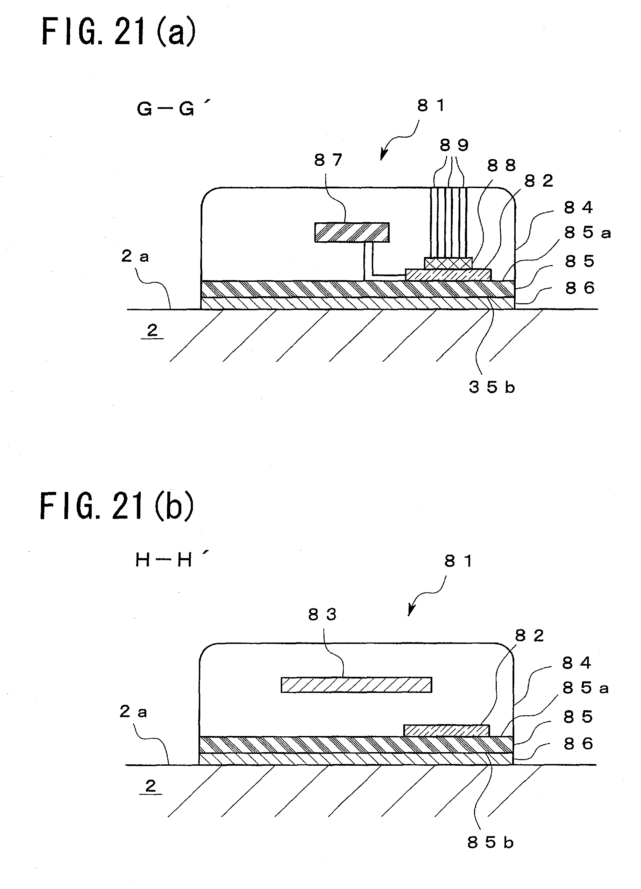

Next, a medical device according to Embodiment 9 is described referring to FIGS. 20 and 21. The medical device according to Embodiment 9 is also configured in the same manner as the medical device according to Embodiment 1, 2, or 3, except that a sheet-like member (sheet member) 81 is differently configured. The configuration of the sheet member is mainly described below.

FIG. 20 is a plan view illustrating a sheet-like member used in Embodiment 9 of the present invention. FIG. 21(a) is a cross-sectional view illustrating a cross section of the sheet-like member taken along the cutting-plane line G-G' in FIG. 20, and FIG. 21(b) is a cross-sectional view illustrating a cross section of the sheet-like member taken along the cutting-plane line H-H' in FIG. 20. It should be noted that in FIGS. 21(a) and 21(b), only the lines that appear in the cross section are shown.

As shown in FIGS. 20, 21 (a), and 21(b), in Embodiment 9, as with Embodiments 1 to 3, the sheet member 81 displays information 4 on a user on one principal surface, with the other principal surface being attached to an outer surface 2a of a main body of the medical device. Moreover, the sheet member 81 includes, as with the sheet member 3 of Embodiment 1 shown in FIG. 2(b), a sheet-like base 85, a protective layer 84 having optical transparency, and an adhesive material layer 86. Then, the protective layer 84 is formed on one principal surface (upper surface) 85a of the base 85, and the adhesive material layer 86 is formed on the other principal surface (lower surface) 85b of the base 85. Thus, the sheet member 81 has the same configuration as the sheet member 3 used in Embodiment 1 except for the following.

As shown in FIGS. 20, 21(a) and 21(b), in Embodiment 9, the sheet member 81 includes a circuit substrate 82, a pressure sensor 87 that detects a pressure added to the protective layer 84, a speaker 88, and a plate 83 on which the information is printed. The circuit substrate 82 is arranged on the one principal surface 85a of the base 85. The pressure sensor 87 is arranged to be suspended within the protective layer 84. The plate 83 is also arranged to be suspended within the protective layer 84 above the circuit substrate 82. It should be noted that the hatching of the protective layer 84 is omitted in FIGS. 21 (a) and 21(b).

The speaker 88 is mounted on the circuit substrate 82. Furthermore, a power supply circuit that supplies power to the speaker 88, a signal processing circuit that drives the speaker 88 based on a signal from the pressure sensor 87, and the like are also mounted on the circuit substrate 82, although these are not shown. The power supply circuit is also provided with a battery (not shown). Moreover, the protective layer 84 is provided with a through hole 89 for outward propagation of a sound outputted from the speaker 88.

In Embodiment 9, the pressure sensor 87 is not particularly limited but may be any pressure sensor that is capable of detecting pressing force applied to the protective layer 84. In Embodiment 9, for example, a diaphragm (not shown) on which a piezoresistive element is formed is used as the pressure sensor 87. In this case, when the protective layer 84 is pressed, and thereby the diaphragm is deformed, the electric resistance of the piezoresistive element changes, and at the same time, the level of the electric signal from the pressure sensor 87 also changes.

The signal processing circuit (not shown) provided for the circuit substrate 82 detects the level of the electric signal from the pressure sensor 87, and thereby decides that the protective layer 84 has been pressed. Then, the signal processing circuit causes the power supply circuit to supply power to the speaker, thereby outputting a predetermined sound from the speaker.

Moreover, by allowing the user to select the sound to be outputted, the user can identify his or her own medical device based on the sound from the sheet member 81. Furthermore, by outputting music from the sheet member 81, the user can be relaxed with the music. Moreover, in particular, when the user is a patient suffering from a disease, the music can be expected to alleviate pain. Furthermore, a medical device to which the sheet member 81 is attached outputs a sound also when the user has dropped the device by accident. Therefore, this is particularly advantageous for the user with hearing impairment or blindness.

In Embodiment 9, the formation materials of the base 6 and the protective layer 5 of Embodiment 1 may be used as the formation materials of the base 85 and the protective layer 84. There is no particular limitation with respect to the formation material, the thickness, and the like of the adhesive material layer 86. Moreover, in Embodiment 9, the plate 83 may employ a mode in which it is in touch with the circuit substrate 82, or may be arranged in an area of the base 85 where the circuit substrate 82 is not arranged. Furthermore, Embodiment 9 may employ a mode in which the sheet member 81 does not include the plate 83. In this case, the information 4 is printed in the area of the base 85 where the circuit substrate 82 is not arranged.

### Industrial Applicability

As described above, the present invention is advantageous, in particular, for a small portable medical device. The medical device according to the present invention has industrial applicability.

## Claims

1. A medical device that measures a condition of a living body, comprising:
a main body containing a measuring device inside; and
a sheet-like member,
the sheet-like member displaying information on a user on one principal surface, with the other principal surface being attached to an outer surface of the main body.

2. The medical device according to claim 1, wherein,
either one or both of a projection and a recess is formed on the one principal surface of the sheet-like member, and
the projection and the recess that are formed express information on operation of the medical device by means of at least one of arrangement, shape, and size thereof.

3. The medical device according to claim 1 or 2, wherein
the sheet-like member includes a storage device that exchanges information with the measuring device.

4. The medical device according to any of claims 1 to 3, wherein
the sheet-like member includes a sheet-like base, a protective layer formed on one principal surface of the base, and an adhesive material layer formed on the other principal surface of the base,
the protective layer being formed of a material having optical transparency.

5. The medical device according to claim 4, wherein
the sheet-like member has granular identification members that are dispersed inside the protective layer,
the identification members being dispersed in such a manner that an area where the identification members exist does not overlap the information in a direction of the normal to the base, and being formed of any of a luminescent material, a thermochromic material, and a light reflective material.

6. The medical device according to claim 4, wherein
the sheet-like member further includes an identification layer between the base and the protective layer,
the identification layer being formed of a luminescent material or a light reflective material.

7. The medical device according to claim 4, wherein
the sheet-like member further includes an identification layer that covers the protective layer,
the identification layer having an opening in a portion that overlaps the information in a thickness direction of the base, and being formed of any of a luminescent material, a thermochromic material, and a light reflective material.

8. The medical device according to claim 4, wherein
the sheet-like member has an electronic circuit that communicates with outside by radio transmission or optical communication.

9. The medical device according to claim 4, wherein
the sheet-like member further includes a fragrance component-impregnated layer containing a fragrance component,
the fragrance component-impregnated layer being arranged above the one principal surface of the base, and
the protective layer being formed so as to cover the fragrance component-impregnated layer and having a vent for diffusing the fragrance component outward.

10. The medical device according to claim 4, wherein
the sheet-like member further includes a pressure sensor that detects a pressure added to the protective layer, a speaker, and a circuit substrate,
the circuit substrate being arranged on the one principal surface of the base, and including a signal processing circuit that drives the speaker based on a signal from the pressure sensor, and
the protective layer being formed so as to cover the circuit substrate, the pressure sensor, and the speaker.
